# EUROPEAN PATENT SPECIFICATION

(11) **EP 2 819 616 B1**
(45) Date of publication and mention of the grant of the patent: **09.10.2019**
(21) Application number: 13755292.3
(22) Date of filing: 28.02.2013
(51) Int. Cl.: A61F 2/00, A61L 31/02, A61L 31/08, D04B 21/00, A61L 31/04, A61L 31/06, A61L 31/14

(54) **TISSUE SUPPORT STRUCTURE**
GEWEBESTÜTZSTRUKTUR
STRUCTURE DE SUPPORT DE TISSU

(30) Priority: 28.02.2012 US 201261603958 P; 27.02.2013 US 201361769767 P
(43) Date of publication of application: 07.01.2015
(73) Proprietor: Incorpracyl Technologies Ltd., 9644009 Jerusalem (IL)
(72) Inventor: GORMAN, Joel, 9644009 Jerusalem (IL)
(74) Representative: Kretschmann, Dennis
(86) International application number: PCT/IL2013/000023
(87) International publication number: WO 2013/128434

(56) References cited:
- EP-A1- 2 340 784
- WO-A1-01/80773
- WO-A1-96/03091
- WO-A1-03/094781
- WO-A2-2010/088699
- DE-A1- 10 353 930
- DE-A1-102008 044 005
- US-A1- 2006 281 967
- US-A1- 2009 024 147
- US-A1- 2010 256 756
- US-A1- 2011 014 153

## Description

### FIELD OF THE DISCLOSED TECHNIQUE

The disclosed technique relates to implantable devices in general, and to implantable devices for the surgical treatment of tissues, in particular.

### BACKGROUND OF THE DISCLOSED TECHNIQUE

In general, a hernia is a protrusion of the internal content of a cavity through a defect in the wall of the cavity. The term "hernia" may also refer to the defect itself, through which the protrusion occurs.

### Relevant Anatomy

In the field of human medicine, hernias most frequently occur in the abdomen, where intra-abdominal content protrudes out through a defect in the abdominal wall. This protruding content might include vital organs, usually parts of the intestine, or intra-abdominal fat, called "omentum". Since such protrusions are common and can lead to serious illness and death, the medical science of treating these protrusions receives ongoing attention and a surgical sub-specialty is entirely devoted to the treatment of hernias of the abdominal wall.

The anterior wall of the human abdomen gets its strength and integrity primarily from three sheet-like muscles which are layered one on top of another and surround the front and sides of the abdomen. The muscle sheets are held together firmly by a facial envelope or "aponeurosis" that becomes notably thicker (approximately 1mm), stronger and adherent to the muscles on the outer surface of this musculo-aponeurotic complex. External to the musculo-aponeurotic complex is the subcutaneous fat and overlaying skin of the abdomen. Internal to this wall-like musculo-aponeurotic complex is the thin lining of the abdominal cavity called the "parietal peritoneum", which generally surrounds the enclosed internal organs.

### Causes of Hernias

Hernial defects occur in the musculo-aponeurotic layer of the abdominal wall either due to birth defects, the "wear and tear" of aging, or inadequately closed old surgical scars. When abdominal content protrudes out through the defect in the abdominal wall, the content is frequently covered by the parietal peritoneum. This peritoneal covering thickens with time, presumably as a pathologic reaction to being out of place within the subcutaneous tissue. The thickened peritoneal covering gradually takes the form of a sac which grows larger as content therein progressively protrudes. This results in the characteristic bulging seen and felt as a hernia under the skin and fat.

### Known Treatment of Hernias

Attempts to permanently correct hernias have been based on restoring the protruding contents back into the abdomen and then surgically closing the defect in the abdominal wall through which the protrusion originally occurred. Since the defect is essentially a hole in the musculo-aponeurotic complex, all earlier attempts (from circa 1880 to 1980) to close the defect relied on the simple method of surgically sewing or "suturing" the defect with a needle and thread. These suturing techniques however exhibited a high rate of reopening of the repair site and recurrence of the hernia. However, the rate of other complications (e.g., chronic pain, chronic infection) remained substantially limited.

More modern attempts of hernia surgery (circa 1960 to the present) were brought about by the post-World War II development of plastics, in the form of meshes, which are used to permanently close abdominal defects. In general, plastic meshes are either fiber-based networks or solid sheets. Known in the art fiber-based meshes are formed by weaving, interlacing, interweaving, knotting, knitting, winding, braiding, entangling or intertwining elongated elements, such as thread sections, which are intersected or substantially affixed with each other to form a network of nodes or intersections. These intersections are separated by holes, openings or pores, typically being evenly spaced. In known in the art fiber-based meshes, the individual plastic fibers are monofilaments, which are either closely intertwined or braided together. The highly braided or intertwined monofilament fibers are then further allied one to another in various patterns, such as matrices or random weaves. The overall result is a rough surface with numerous intersections, and numerous substantially small crevices or nooks-and-crannies at the macroscopic and microscopic levels. The applicant estimates the number of intersections per 100 square centimeters to be on the order of hundreds of thousands of intersections with the resulting crevices. The terms "fiber intersection" or "intersection" herein relate to two fiber sections firmly coupled with each other so as to prevent tissue growth therebetween. Unlike fiber-base meshes, most known in the art plastic solid sheet meshes exhibit micron-porosity, (i.e., the diameter of the pores is on the order of micrometers).

In general, plastic meshes are employed as implantable permanent barriers or tissue reinforcing structures. The effective functioning of plastic meshes depends on the bio-compatibility (i.e., inertness) of plastic and on the permanent incorporability of the plastic mesh into the surrounding tissues. The term "incorporable" relates to the structure being able to be fully incorporated into the tissue. Regarding incorporability, it is known in the art that an inflammatory tissue reaction is required for mesh incorporation to occur. In other words, for incorporation of the mesh into the tissue to occur, the mesh must stimulate the ingrowth of reactive tissue to attach to and surround the individual or groups of mesh fibers and thus fix the mesh permanently in place.

However, on a practical level, meshes have also introduced new mesh-related complications to hernia repair. These mesh related complications are, for example, chronic mesh pain, chronic mesh infection and significant visceral adhesions to the mesh. The relationship between the requirement of tissue reactivity for incorporability and mesh-related complications eventually became recognized. Generally, known in the art fiber-based meshes engender an inflammatory response other than the reaction required for tissue incorporation alone. This inflammatory response may be a detrimental side effect causing complications related to fiber-based meshes. Conversely, known in the art solid sheet meshes, which are non-reactive and do not allow for tissue attachment and ingrowth, are largely non-incorporable. This non-incorporability of solid sheet meshes causes the complications related to the solid sheet mesh.

### The Detailed Causes of Complications of Current Mesh Surgery

The drawbacks of current surgery of abdominal wall hernias using meshes are chronic mesh pain syndromes (e.g., foreign-body sensation, stiff-abdomen syndrome), chronic infection of the mesh, complications due to visceral adhesions to the mesh and hernia recurrence. Generally, the underlying cause common to the complications of known in the art fiber-based meshes is the inflammatory reaction of the tissues to the intersecting fibers. As mentioned above, in known fiber-based meshes, monofilament threads are closely intertwined or braided together and further allied one onto another. This results in a rough surface which includes a substantial number of nooks and crevices. These nooks and crevices cause the inflammatory response to proliferate and persist, which eventually results in the formation of primitive disorganized scar tissue. While the formation of such scar tissue may lead to the incorporation of the fiber-based mesh into the tissues, the scar tissue is also an uncontrolled foreign-body reaction, which may cause the mesh to stiffen and shrink by up to 40% of the mesh's original size. The stiffening of the mesh leads in turn to chronic mesh pain syndromes. The shrinkage of the mesh may cause the mesh to disattach from the tissues and migrate, leading to hernia recurrence. Moreover, the substantial growth of inflammatory tissue engendered by the crevices encourages adhesion formation between viscera and the mesh, causing bowel obstruction and enterocutaneous fistula. Also, the dense mass of the combined scar tissue and mesh may contribute to the sequestering of chronic infections.

The cause common to the complications of known in the art solid sheet meshes is the prevention of tissue ingrowth (i.e., either normal or inflammatory), which in turn prevents mesh incorporation. As a result the mesh is liable to migrate and cause hernia recurrence. Solid sheet meshes tend to become separated from the abdominal wall and walled-off or encapsulated, typical of a foreign body. This encourages chronic infections, which is characteristic of this type of mesh. In addition, the walled-off non-incorporated solid sheet acts as a permanent irritant causing significant chronic pain.

### Known in the Art Methods to Decrease Mesh Complications

While the inflammatory or foreign-body reactions described above are essentially unavoidable following implantation of all known in the art fiber-based or solid sheet meshes, attempts have been made to cause the body to react differently to fiber-based meshes by manipulating either the placement position of the mesh or the material composition of the mesh.

Regarding placement position of the mesh, there are generally two known options. One option consists of external or "open" placement onto or into the abdominal wall via a large incision under direct vision. The other option consists of intra-abdominal placement via video-directed laparoscopy. With both options there are various ways for positioning the mesh inside the body, each way exhibiting differing effects on the complications described above. The external option is less associated with intra-abdominal visceral adhesions, but more associated with chronic infections. Conversely, laparoscopic placement increases the likelihood of complications from visceral adhesions but reduces the risk of mesh infection. Neither of these options substantially alters the high rate of chronic pain syndromes.

Regarding the options of placement positions using the external approach for minimizing complications, externally placed meshes can be put in the following positions: "onlay", "inlay", "sublay" or "intraperitoneal". Onlay positioning is considered simple to execute and substantially eliminates the risk of visceral adhesions. However a substantially high rate of chronic infections is still observed. Inlay positioning consists of attaching the mesh to the borders of the hernia defect. Regardless of any advantages afforded thereby, the inlay position generally exhibits a high rate of hernia recurrence. Sublay positioning, which may be associated with operative complications, reduces mesh infection and hernia recurrence, but stiff-abdomen and other chronic pain syndromes are not avoided. Intraperitoneal positioning also exhibits a high rate of visceral adhesions when using known in the art fiber-based meshes.

Regarding laparoscopic placement, two types of placements using known in the art meshes are common, namely total intra-abdominal placement and preperitoneal placement. With known in the art fiber-based meshes, the intra-abdominal placement exhibits a high degree of complications, especially visceral adhesions and pain. Furthermore, intra-abdominal laparoscopic placed meshes are noted for being difficult to remove when necessary. Preperitoneal placement supposedly minimizes visceral adhesions and mesh infection, but does not minimize pain sequelae. In addition, preperitoneal placement is considered a difficult surgical procedure to perform.

As mentioned above, altering the material composition of fiber-based meshes has been attempted to reduce mesh-related complications. These attempts have been based on the recognition that the inflammatory response to fiber-based compositions is the direct cause of most of the complications, as described above. Accordingly, attempts have been made to reduce the amount of inciting material in the mesh, for example, by using less material or by increasing the pore size between the braided or intertwined monofilament fibers. However, these material alterations still stimulate the proliferation of the inflammatory tissue response when being incorporated. Results from recent experiments using very large pore meshes of up to 3.6 millimeters pore diameter have not lowered complications significantly.

Alternative attempts have been made to control the inflammatory reaction by using non-synthetic biological materials, such as cross-linked or non-cross-linked acellular dermis of porcine or human origin. Biological meshes have a common property of being dissolved or absorbed by the inflammatory reactions of the tissues that they are meant to reinforce. As such, these biological meshes are generally precluded from the primary permanent treatment of abdominal wall hernias. Nevertheless, they have two potential roles in abdominal wall surgery. First, when joined to a permanent synthetic mesh as a composite mesh, the biological mesh is meant to protect the synthetic mesh from visceral adhesions as the biological mesh gradually disappears. In practice however, adhesions are still frequent and cause a high rate of complications. Furthermore, when the biological mesh is absorbed, the permanent synthetic mesh may still cause chronic pain syndromes. Secondly, biological meshes have proven very useful and often life-saving in "catastrophic" abdomen settings (e.g., a highly contaminated wide open abdomen), where permanent synthetic mesh is contraindicated due to the high probability of chronic mesh infection. A fully absorbed biological mesh may safely provide the necessary covering for the time period required for any potential source of contamination to resolve and for a definitive repair to be carried out. Unfortunately, the high cost of biological meshes is a major drawback in their use. In light of the above, it is apparent that the property which determines the effectiveness of known in the art meshes in repairing hernia defects, namely the reactivity of the mesh, is the same property that leads to mesh complications of current fiber-based and solid sheets meshes, namely infection, adhesions, pain and recurrence. Furthermore, the reactivity of biological meshes is the cause for absorption and eventual disappearance, leading to hernia recurrences.

U.S. Patent Application Publication No. 2009/0024147 to Ralph et al., entitled "Implantable Mesh for Musculoskeletal Trauma, Orthopedic Reconstruction And Soft Tissue Repair" is directed to implantable structures for the treatment of musculoskeletal trauma, orthopedic reconstruction and soft tissue applications made of biocompatible mesh materials. The implantable structure of Ralph et al. includes two perpendicular sets of strands crossed over and under each other in an alternating pattern that intersects at points of contact. The spacing between the strands may be configured to produce a less permeable mesh with smaller voids or may be configured to tailor the voids (e.g., larger, smaller or variable spacing) according to hard or soft tissue ingrowth requirements. The strand material can be any biocompatible implant material such as metallic, bioresorbable polymers and non-resorbable polymers as well as organic materials such as collagen. The strands may exhibit various physical structures. For example, they can exhibit monofilament, thread or yarn structures. They can be braided or they can be hollow tubular structures and the hollow tubular structures can have a cross-section which is round, oval, square, rectangular, triangular or of any other closed geometric shape, including irregular shapes. When the hollow strands are either porous or biodegradable, the strands may be filled with medication or bone growth substances to provide a timed release at the surgical site.

US2006281967 discloses in par. 0010 to relate to a prosthetic openwork knit for the treatment of urinary incontinence and/or prolapse, based on an arrangement of yarns of a biocompatible polymer comprising at least one first sheet defining a first chain structure, in which knit said arrangement of yarns further comprises at least two non-meshing sheets, of partial weft, the number of chain yarns in said chain structure being from 6 to 12.

DE 103 53 930 A1 discloses in paragraphs 25-27 and 33 a textile intraperitoneal mesh that comprises monofilaments that form pores of size less than 2 mm.

WO 96/03091 A1 discloses a surgical mesh implant that has minimal pore size of 0.5 mm and maximal pore size of 10 mm, preferably 1.5 to 4 mm. The 10 mm maximal pore size represents a maximal pore area of 100 mm² (i.e. 10 mm multiplied by 10 mm). According to WO 96/03091, the maximal porosity of the surgical mesh is determined according to the forces that the mesh is required to resist, and the pore sizes are determind according to the understanding and knowledge of the art and therefore the area of the pores is limited to a maximum of 100 mm².

### SUMMARY OF THE PRESENT DISCLOSED TECHNIQUE

It is an object of the disclosed technique to provide a novel implantable tissue support structure for supporting tissue. In accordance with the disclosed technique, there is thus provided a tissue support structure which comprises a plurality of non-braided monofilament thread sections which define a surface. The tissue support structure induces substantially no foreign body reaction when supporting the tissue other than the reaction associated with the healing of tissue being in contact with a single non-braided monofilament thread and allows for substantially unimpeded ingrowth of healing tissue, by maintaining the number of monofilament thread intersections to be smaller than 10,000 intersections per one-hundred square centimeters. The thread intersection are defined as the crossing of two of the thread sections resulting from at least one of braiding, weaving, entangling, intertwining and affixing the thread sections.

### BRIEF DESCRIPTION OF THE DRAWINGS

The disclosed technique will be understood and appreciated more fully from the following detailed description taken in conjunction with the drawings in which:
Figure 1A is a schematic illustration of an implantable, incorporable and non-reactive tissue support structure exhibiting the "parallel lines" configuration, constructed and operative in accordance with an embodiment of the disclosed technique;
Figures 1B and 1C are schematic illustrations of implantable, incorporable and non-reactive tissue support arrays, constructed from tissue structures each exhibiting the "parallel lines" configuration in accordance with another embodiment of the disclosed technique;
Figures 2A-2F are schematic illustrations of parallel lines, implantable, incorporable and non-reactive tissue support structures and tissue support structure arrays, with a stabilizing perimeter structure, constructed and operative in accordance with a further embodiment of the disclosed technique;
Figure 2G is a schematic illustration of a tissue support structure constructed and operative in accordance with another embodiment of the disclosed technique.
Figures 3A-3D are schematic illustrations of an implantable, incorporable and non-reactive tissue support structure exhibiting the matrix configuration, constructed and operative in accordance with a further embodiment of the disclosed technique;
Figures 4A-4B are schematic illustrations of an implantable, incorporable and non-reactive tissue support structure exhibiting the randomly intersecting lines configuration, constructed and operative in accordance with another embodiment of the disclosed technique;
Figures 5A-5B are schematic illustrations of an implantable, incorporable and non-reactive tissue support structure exhibiting the hub and radiating spokes configuration, constructed and operative in accordance with a further embodiment of the disclosed technique;
Figures 5C-5D, which are schematic illustrations of an implantable, incorporable and non-reactive tissue support structure exhibiting a hub and radiating spokes configuration with concentric rings, constructed and operative in accordance with another embodiment of the disclosed technique;
Figure 5E, which is a schematic illustration of an implantable, incorporable and non-reactive tissue support structure array made of a plurality of hub with radiating spokes tissue support structures, constructed and operative in accordance with a further embodiment of the disclosed technique;
Figures 6A-6B is schematic illustration of an implantable, incorporable and non-reactive tissue support structure array made of quilted hubs with radiating spokes tissue support structures, constructed and operative in accordance with another embodiment of the disclosed technique;
Figures 7A-7D are schematic illustrations of implantable, incorporable and non-reactive tissue support structures exhibiting invertible convexity, constructed and operative in accordance with a further embodiment of the disclosed technique;
Figures 8A-8D are schematic illustrations of different exemplary cross-sections of a stabilizing perimeter structure constructed and operative in accordance with another embodiment of the disclosed technique;
Figures 9A and 9B are schematic illustrations of a section of an implantable, incorporable and non-reactive tissue support structure constructed and operative in accordance with a further embodiment of the disclosed technique;
Figure 10 is a schematic illustration of a section of an implantable, incorporable and non-reactive tissue support structure, constructed and operative in accordance with another embodiment of the disclosed technique;
Figure 11 is a schematic illustration of a tissue support structure, exhibiting the matrix configuration, constructed and operative in accordance with a further embodiments of the disclosed technique; and
Figures 12A - 12C are schematic illustrations of a tissue support structure constructed and operative in accordance with another embodiment of the disclosed technique.
Figures 13A-13G, are schematic illustrations of cross-sections of various stabilizing perimeter structures, constructed and operative in accordance with a further embodiment of the disclosed technique;

### DETAILED DESCRIPTION OF THE EMBODIMENTS

The disclosed technique overcomes the disadvantages of the prior art by providing a tissue support structure for medical applications, for example, for the treatment of hernias of the abdominal wall. The tissue support structure according to the disclosed technique is an implantable, non-reactive and incorporable structure which is made of a singular non-braided monofilament thread or threads having between 0 and up to 10,000 thread intersections per 100 square centimeters (cm²). In other words, the tissue support structure according to the disclosed technique is made of singular non-braided monofilament thread sections which induce substantially no foreign body reaction other than the reaction associated with the healing of tissue being in contact with a single non-braided monofilament thread when supporting said tissue. Furthermore, the tissue support structure according to the disclosed technique allows for substantially unimpeded ingrowth of healing tissue (i.e., healing tissue which is associated with the healing of tissue being in contact with a single non-braided monofilament thread), by maintaining the number of monofilament thread intersections to be smaller than 10,000 intersections per 100 cm².

A tissue support structure made of singular non-braided monofilament thread or threads may be stabilized by at least one stabilizing method or structure. The number of thread intersections per 100 cm² is referred to herein as the "thread intersection density" or just the "intersection density". In addition the term "thread" and the term "fiber" are used herein interchangeably. The term "non-reactive" above relates to the fact that the tissue implanted with the tissue support structure of the disclosed technique does not exhibit a substantial foreign body reaction to the implanted structure other than the tissue reaction associated with healing tissue being in contact with a single non-braided monofilament thread. As mentioned above, the term "incorporable" relates to the ability of the structure to be fully incorporated into the tissue. Thus, the tissue support structure according to the disclosed technique is fully incorporable into the tissue, without causing substantial tissue reaction (i.e., being non-reactive).

As mentioned above, the tissue support structures according to the disclosed technique are constructed from a singular non-braided monofilament thread or thread sections. In some embodiments, the thread sections do not intersect with each other (e.g., they are laid in parallel). In other embodiments of the disclosed technique, sections of the threads intersect with each other, thus creating a network. The tissue support structure may be stabilized, for example, by affixing the thread intersections at least at one point, by a stabilizing perimeter structure or by a central hub. The term "stabilizing" herein refers to maintaining the structure or the thread sections relative to each other. Thus, the terms "stabilized" or "structurally stabilized" herein refer to the fact that the structure or the fiber sections relative to each other is maintained. The singular non-braided monofilament threads typically consist of nylon or other synthetic materials (e.g., metals, carbons and polytetrafluoroethylene). The threads may have a diameter of 0.2 mm or more. According to one embodiment the threads have a diameter of 0.35 mm. The threads may exhibit a circular cross-section, a rectangular cross-section or any other geometrical shape. Each thread may include protrusions (e.g., spherical bead-like bulges or cubical bulges), positioned intermittently along the thread at regular or irregular intervals therebetween. The size of each such protrusion may be up to one centimeter in diameter or as its longest side. Tissue support structures according to the disclosed technique may be coupled together, as further explained below, to create a tissue support structure array.

The tissue support structure according to the disclosed technique may be used by applying the structure to hernia defects according to any of the methods of applying meshes, such as laparoscopically, or by non-laparoscopic open surgery using, for example, "onlay" or "sublay" positioning, as explained above. The tissue support structure according to the disclosed technique is attached to the musculo-aponeurotic complex with, for example, sutures, clips or biocompatible adhesives.

Five configurations of the disclosed technique shall be exemplified herein below and described with reference to the figures. These five configurations are the parallel lines configuration, the matrix configuration, the randomly intersecting lines configuration, the radiating spokes with concentric rings configuration and the "quilted" hub and radiating spokes configuration. All of the described configurations are made of non-braided monofilament threads resulting in an implantable incorporate and non-reactive tissue support structure.

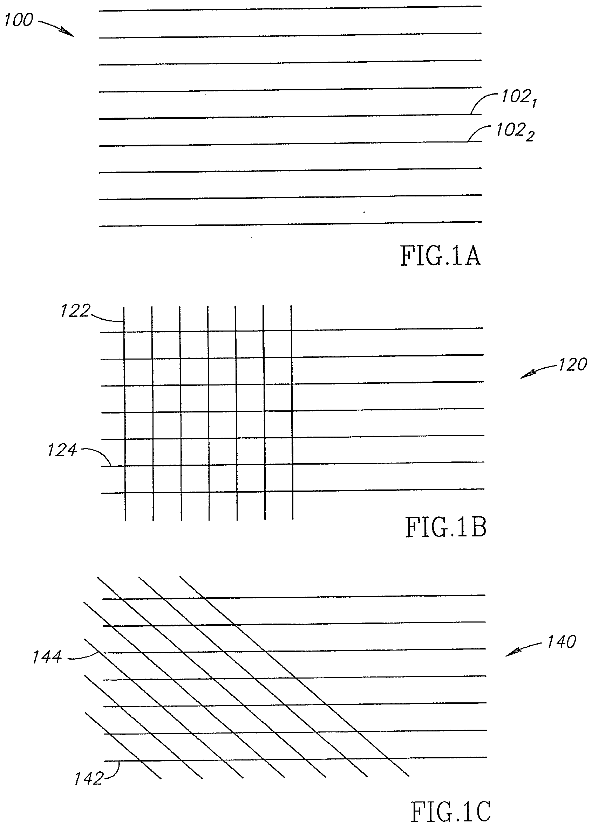

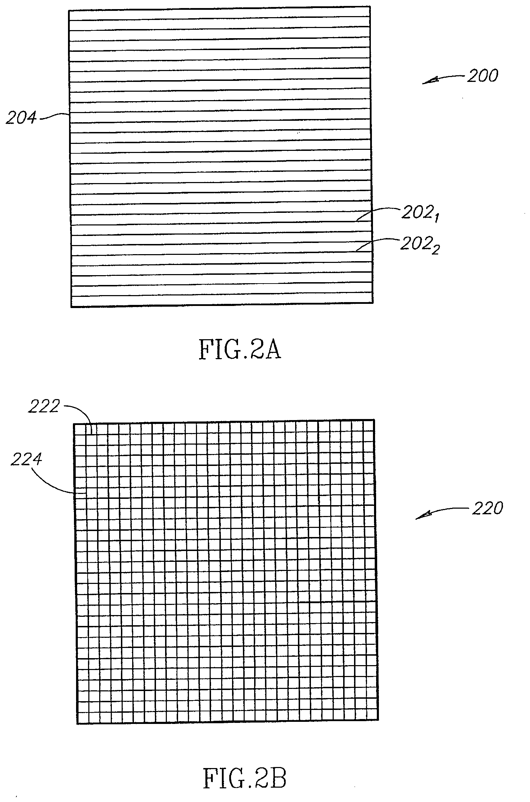

The "parallel lines" configuration is illustrated in Figures 1A-1C and 2A-2G. Reference is now made to Figure 1A, which is a schematic illustration of an implantable, incorporable and non-reactive tissue support structure exhibiting the "parallel lines" configuration, generally referenced 100, constructed and operative in accordance with an embodiment of the disclosed technique. Tissue support structure 100 includes parallel sections of singular, non-braided, monofilament threads, for example, threads 102₁ and 102₂. Thread 102₁ and 102₂ may be a #0 (i.e., 0.35 millimeters in diameter) monofilament nylon thread. It is noted that tissue support structure 100 requires a stabilizing perimeter structure (not shown) as further explained below. The terms "stabilizing perimeter structure", "perimeter structure" and "frame" are herein used interchangeably. Stabilizing perimeter structures are further explained below.

In general, a single layer of parallel threads depicted in Figure 1A may serve as an implantable, incorporable and non-reactive tissue support structure. However, more than one layer placed one on top of the other at various angles may also be employed. Reference is now made to Figures 1B and 1C which are schematic illustrations of implantable, incorporable and non-reactive tissue support arrays, generally referenced 120 and 140 respectively, constructed from tissue structures each exhibiting the "parallel lines" configuration of Figure 1A, constructed and operative in accordance with another embodiment of the disclosed technique. In Figure 1B, a first layer 122 is laid over a second layer 124 such that the threads of the two layers are at right angles with respect to one another. In Figure 1C, a first layer 142 is laid over a second layer 144 such that the threads of the two layers are at non-right angles with respect to one another. The distance between adjacent parallel lines (i.e., threads) may be, for example, 0.5 cm 1.0 cm, 2.0 cm or 3.0 cm.

Placing one layer on top of the other at various angles enables a surgeon to choose the required configuration (i.e., either a parallel lines configuration or an orderly intersecting configuration) according to real-time intra-operative considerations. It is noted that the non-layered parallel lines configuration provides a tissue support structure which contains a minimum of material and intersections (i.e., zero) per 100 cm² of surface area.

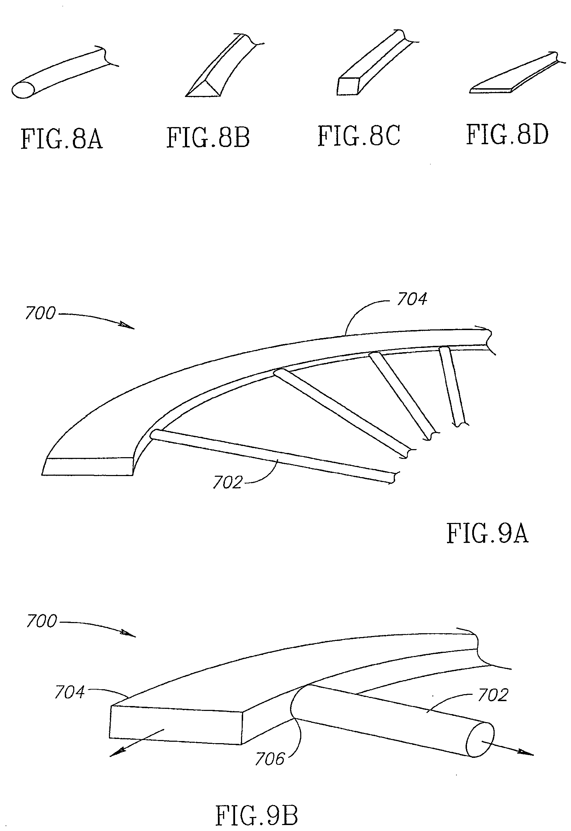

Tissue support structures exhibiting the parallel lines configuration may be stabilized with a stabilizing perimeter structure. Stabilizing perimeter structures are further explained below in Figures 8A-8D, 9A-9B and 10. Parallel lines tissue support structures having such a stabilizing perimeter structure are schematically illustrated in Figures 2A-2F. Reference is now made to Figures 2A-2F, which are schematic illustrations of parallel lines, implantable, incorporable and non-reactive tissue support structures and tissue support structure arrays, with a stabilizing perimeter structure, generally referenced 200, 220, 240, 260, 280 and 300 respectively, constructed and operative in accordance with a further embodiment of the disclosed technique. In Figure 2A, tissue support structure 200 includes a plurality of parallel singular monofilament threads such as threads 202₁ and 202₂, all connected at both ends to a stabilizing perimeter structure 204. Stabilizing perimeter structure 204 may be made of nylon, metals, carbon amalgams, silicon, polytetrafluoroethylene (PTFE - also known commercially as Teflon®) or other inert materials.

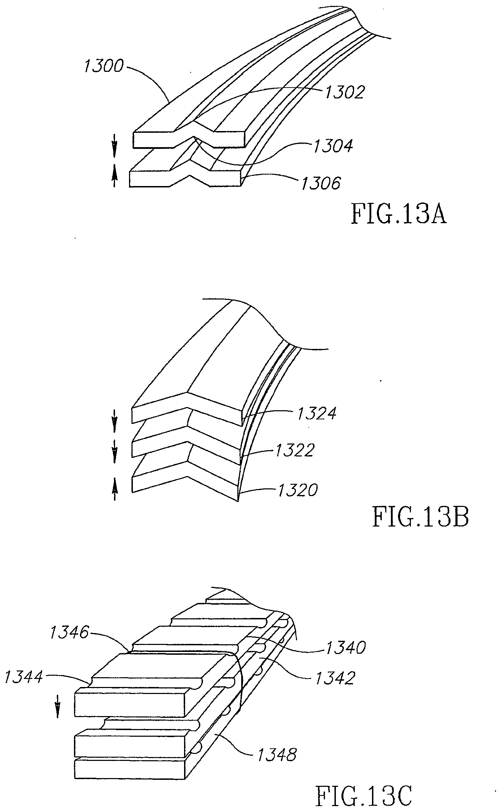

In Figure 2B, a parallel lines tissue support structure array 220 with a stabilizing perimeter structure is made of two separate parallel lines structures, a first parallel lines structure 222 and a second parallel lines structure 224, each similar to tissue support structure 200 (Figure 2A). Second structure 224 is laid on top of first structure 222 such that the parallel threads of first structure 222 are at a right angle with respect to the parallel threads second structure 224. Structure 222 has "vertical" parallel lines and structure 224 has "horizontal" parallel lines resulting in a matrix-like tissue support structure array. The combined stabilizing perimeter structure of tissue support structure 220 is composed of the respective stabilizing perimeter structures of tissue support structure 222 and of tissue support structure 224 coupled together. The coupling of different stabilizing perimeter structures is further explained below in conjunction with Figures 13A-13G.

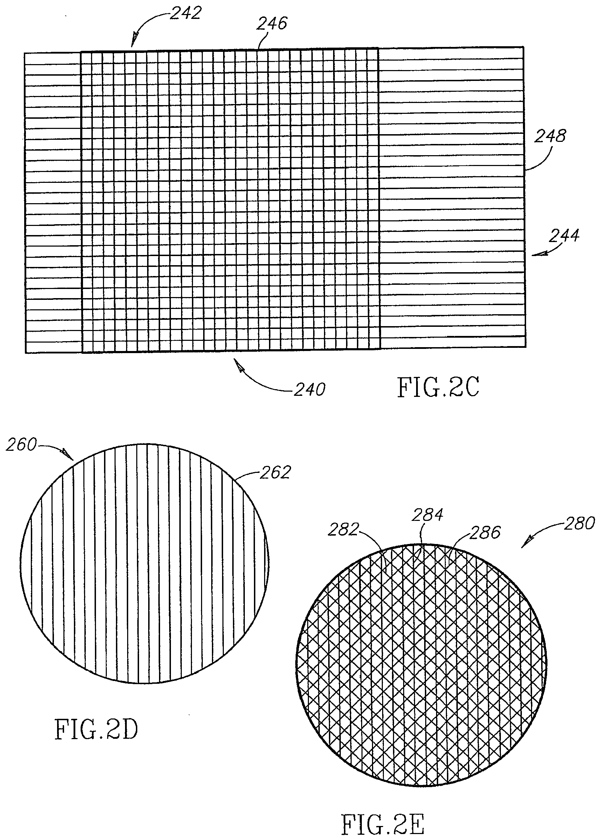

The two or more overlaying structures do not necessarily exhibit identical shapes and sizes. For example, as depicted in Figure 2C, a parallel line tissue support structure array 240 is made of two separate parallel lines structures, a first parallel lines structure 242 and a second parallel lines structure 244, each similar to tissue support structure 200 (Figure 2A). First parallel lines structure 242 is laid over second parallel lines structure 244. First parallel lines structure 242 includes a square stabilizing perimeter structure 246 and second parallel lines structure 244 includes a rectangular stabilizing perimeter structure 248. First parallel lines structure 242 may exhibit the dimensions of, for example, 10×20 cm or 20×30 cm. Second parallel lines structure 244 may exhibit the dimensions of, for example, 10×10 cm or 20×20 cm.

Figure 2D depicts a parallel lines tissue support structure 260 with a circular, ring shaped, stabilizing perimeter structure 262. In Figure 2E, tissue support structure array 280 is made of three circular structures 282, 284 and 286, each similar to tissue support structure 260 (Figure 2D). The three circular structures 282, 284 and 286 are laid one on top of another such that the angles between the parallel threads of each of tissue support structure 282, 284 and 286 are non-right angles (e.g., 30 degrees, 60 degrees and the like).

In general, any number of layers may be placed at any appropriate angle with respect to one another, to create during surgery a multi-layered implantable, incorporable and non-reactive tissue support structure array. As mentioned above, when only one tissue support structure which exhibits the parallel lines configuration is used the intersection density is at a minimum (i.e., zero per 100 cm²).

The shape of a parallel lines tissue support structure, as defined by the stabilizing perimeter structure thereof, is related to the intended use of the tissue support structure. For example, when the intended purpose is to reinforce a large area of the abdominal wall (i.e., placed over an already mended defect in the abdominal wall), a rectangular shaped parallel lines tissue support structure may be used. Such a rectangular shape parallel lines tissue support structure will extend across the abdominal wall with the minimum number of intersections (i.e., zero per 100 cm²) thus rendering the tissue support structure non-reactive. When the intended use is to re-establish the barrier function of the abdominal wall (i.e., to actually bridge and thus mend a defect in the abdominal wall), two or more parallel line tissue support structures may be overlaid one on top of the other, as explained above with reference to Figures 2B, 2C and 2E.

The circular parallel lines tissue support structure further extends the options of building a tissue support structure during surgery. This is achieved by overlaying more than one identical circular structure, attached at their perimeters. A circular tissue support structure may come in various sizes. Examples of possible sizes include structures with a diameter of 5, 10, 15 or 25 cm.

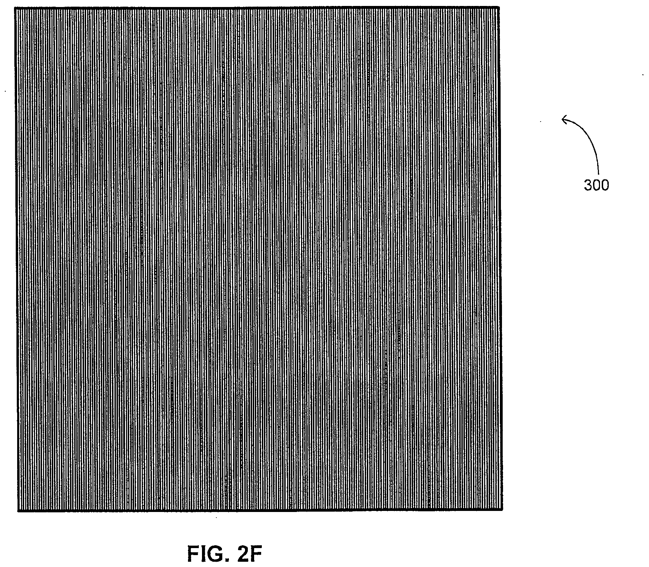

With reference to Figure 2F, tissue support structure 300 is similar to a parallel line tissue support structure (e.g., tissue support structure 200 in Figure 2A) with a stabilizing perimeter structure. The threads in tissue support structure 300 are non-braided monofilament threads, each exhibiting a diameter of at least 10 micrometers (i.e., the diameter of the threads is not necessarily uniform) and are placed in parallel one with respect to the other. Each thread may be in loose parallel contact with the threads adjacent thereto (i.e., adjacent parallel threads are in contact with each other but are not affixed to each other). Thus, tissue support structure 300 may exhibit similar characteristics to that of a solid surface. Alternatively, adjacent pairs of threads may exhibit an intervening space therebetween. In yet another alternative, a first portion of the threads are in loose parallel contact with each other and a second portion of the threads exhibit an intervening space therebetween.

The tissue support structure 300 may be constructed with predefined tautness of the threads. Thus, a tradeoff between compliance of the threads sections to tissue ingrowth and the barrier and re-enforcement function of tissue support structure 300 is achieved. Additionally, tissue support structure 300 may be constructed with each thread section exhibiting a respective tension. Nevertheless, even when adjacent threads are in contact with each other, the threads may be separated when opposing physiological forces are applied to the adjacent threads in the lateral direction. In this way, tissue support structure 300 allows unimpeded tissue ingrowth (i.e., since the thread sections shall move as the healing tissue grows), such as occurs in normal healing, between the threads. Thus, tissue support structure 300 is fully incorporable. Tissue support structure 300 simultaneously exhibits characteristics of a non-reactive solid sheet (i.e., due to the use of singular non-braided monofilament threads with zero intersection density) and a fully incorporable tissue support structure (i.e., due to the loose contact between the thread sections). Such a tissue support structure may by layered upon identical or other tissue support structure described herein. In Figure 2F, tissue support structure 300 exhibits the shape of a square. However, tissue support structure may exhibit various other shapes such as a rectangular, a circle or an ellipse. The size of tissue support structure 300 may exhibit various sizes (e.g., 2 cm or 30 cm as its longest side or diameter).

In the parallel lines configuration, the end parallel threads may be attached to stabilizing parallel rods, which are perpendicular to the threads. A surgeon can control these stabilizing parallel rods with regards to the tautness of the threads (e.g., by holding the two rods apart thus creating the desired tautness). Such a tissue support structure may be used in a similar way to any of the parallel lines configuration described above (e.g., being rolled for insertion via a laparoscope).

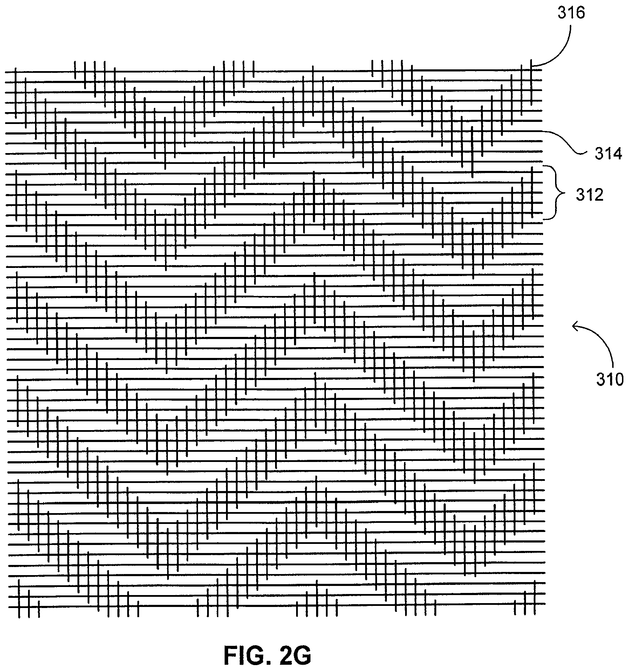

Another method for stabilizing a tissue support structure exhibiting the parallel lines configuration is periodically coupling supporting thread sections across a group of a selected number of threads (i.e., it could be across all of the threads of the tissue support structure). In such cases, the number of intersections is greater than zero but still substantially small (i.e., the intersection density is below 10,000). Reference is now made to Figure 2G, which is a schematic illustration of a tissue support structure, generally referenced 310, constructed and operative in accordance with another embodiment of the disclosed technique. Tissue support structure 310 includes a plurality of parallel non-braided monofilament threads, such as thread 314. At least two threads in each group of a selected number of threads, such as exemplary group 312 (e.g., which includes five threads) is coupled with a supporting thread section, such as supporting thread sections 316. For example, group 312 is affixed to supporting thread sections (e.g., by molding or sintering). Supporting thread section 316 is also made of a non-braided monofilament thread. These support thread sections provide structural support to the parallel threads without substantially increasing the number of thread intersections.

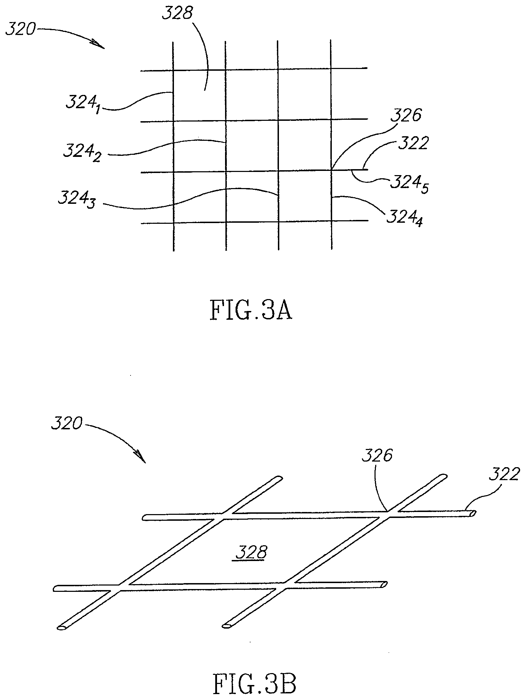

The matrix configuration, as mentioned above, is described herein with reference to Figures 3A-3D, to which reference is now made. Figures 3A-3D are schematic illustrations of an implantable, incorporable and non-reactive tissue support structure exhibiting the matrix configuration, generally referenced 320, constructed and operative in accordance with a further embodiment of the disclosed technique. Tissue support structure 320 includes a network of singular non-braided monofilament threads, such as non-braided monofilament thread 322. The singular non-braided monofilament threads are configured such that sections of a portion of the threads are parallel to and equidistant from sections of other threads (e.g., sections 324₁, 324₂, 324₃ and 324₄). Furthermore, sections of the remaining portions of the thread cross sections of other threads (e.g., section 324₅ crosses sections 324₁, 324₂, 324₃ and 324₄) at intersections such as intersection 326. An intersection is defined as the crossing of two thread sections, which are non-affixed, loosely affixed or firmly affixed at their crossing. Thus, the intersecting thread sections define a plurality of spaces, such as space 328, which exhibit substantially the shape of a square. Thread 322 is a wide bore thread. For example, thread 322 may be a #0 (e.g., 0.35 millimeters in diameter) non-braided monofilament nylon thread (e.g., polyamide 6 or similar material).

Figure 3B depicts a blown up perspective view of a single space 328. Thread 322 is attached to sections of other threads at intersections such as intersection 326. In Figure 3B, the thread sections are firmly affixed by molding the thread sections. However, it is noted that the thread sections may be fused or otherwise affixed in an end-to-side, right-angled, grid-like fashion at regularly spaced intersections. The thread 322 may be fused at intersection 326 by an initial molding process or by heat or other known methods for fusing threads, such as nylon threads.

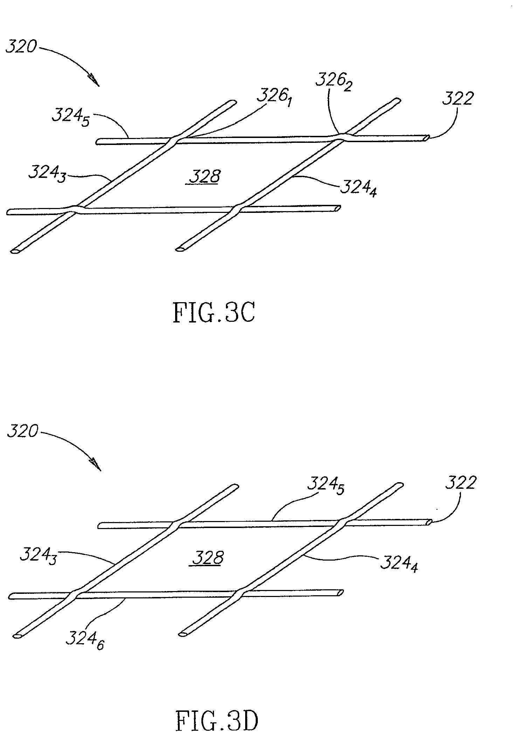

Figure 3C depicts another blown up perspective view of space 328. In figure 3C, the sections of thread 322 are woven into a regularly spaced matrix form in which "horizontal" section 324₅ (i.e., the weft) overlays a "vertical" section 324₄ (i.e., the warp) at one intersection 326₂ but lies under the next "vertical" section 324₃ at the next intersection 326₁. Figure 3D depicts space 328 where "vertical" sections 324₃ and 324₄ are laid upon the "horizontal" sections 324₅ and 324₆ or vice versa without weaving. The "vertical" sections are simply placed on top of the "horizontal" sections.

The inner dimensions of the repetitive square matrix spaces, such as space 328, described hereinabove in conjunction with Figures 3A-3D (i.e., excluding the dimensions of thread 322) are, for example, 0.5×0.5 cm, 0.8×0.8 cm, 2.0×2.0 cm or 3.0x3.0 cm. Other sizes are possible. The spaces need not necessarily be perfect, rigid squares. The aforementioned dimensions of the squares result in an intersection density of only 400, 160, 25 and 10, respectively.

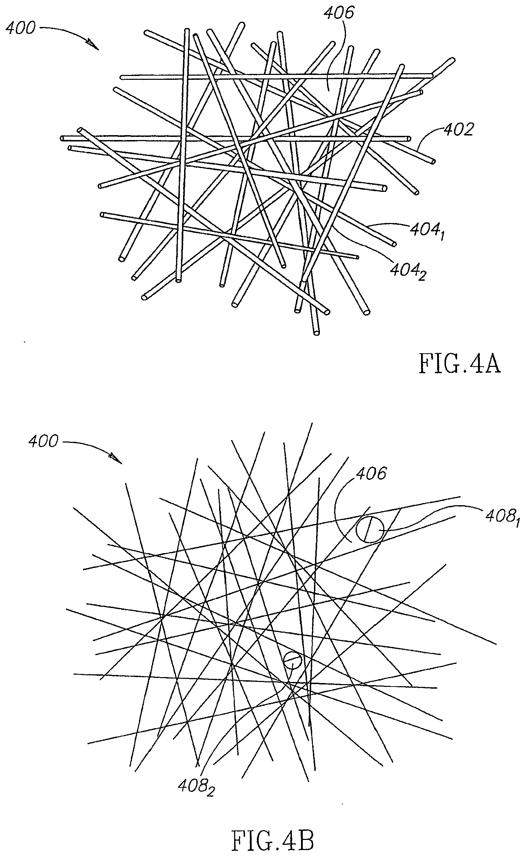

The randomly intersecting threads configuration, as mentioned above, is schematically illustrated in Figures 4A-4B. Reference is now made to Figures 4A-4B, which are schematic illustrations of an implantable, incorporable and non-reactive tissue support structure exhibiting the randomly intersecting lines configuration, generally referenced 400, constructed and operative in accordance with another embodiment of the disclosed technique. Tissue support structure 400 includes a singular, non-braided monofilament thread 402, arranged such that sections of the thread (e.g., sections 404₁ and 404₂) randomly overlay or underlay other sections of the thread. The end result appears as if the sections of thread were randomly woven. The thread sections may be firmly coupled with each other at the intersection point thereof as described above. With reference to Figure 4B, the randomly intersecting sections of the thread produce a plurality of spaces, such as space 406. Tissue support structure 400 may be defined by the maximal and average dimensions of the spaces. For example, in a structure with small spaces, the largest space shall admit therethrough an imaginary circle 408₁ of no more than approximately 1.0 cm diameter, and the estimated average space shall admit therethrough an imaginary circle 408₂ of approximately 0.5 cm diameter. In a structure with larger spaces, the imaginary circles may be 4.0 cm and 2.0 cm in diameter for the largest and average spaces, respectively. Other dimensions and other shapes are possible.

It is noted that the tissue support structure according to the disclosed technique includes between 0 and 10,000 thread intersections per 100 cm² irrespective of the size or shape of the structure. Furthermore, It is also noted that any of the configurations of the disclosed technique (e.g., randomly intersecting lines) may also include a stabilizing perimeter structure as described above with reference to Figures 2A-2F and further elaborated below.

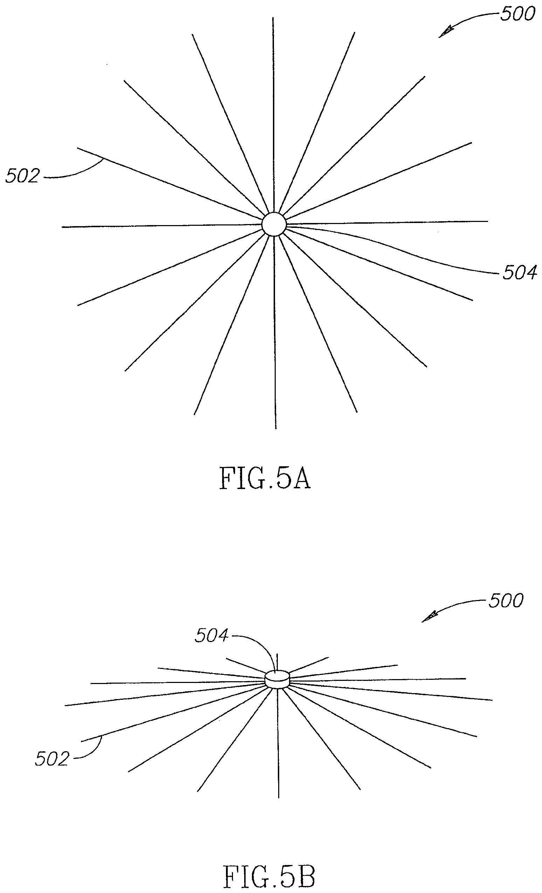

A tissue support structure, exhibiting the form of a hub and radiating spokes and concentric rings, is schematically illustrated in Figures 5A-5D. Reference is now made to Figures 5A-5B, which are schematic illustrations of an implantable, incorporable and non-reactive tissue support structure exhibiting the hub and radiating spokes configuration, generally reference 500, constructed and operative in accordance with a further embodiment of the disclosed technique. Figure 5A depicts a top view of tissue support structure 500 and Figure 5B is a perspective view of tissue support structure 500. Figures 5A and 5B depict tissue support structure 500 (e.g., for hernia repair), which includes a plurality of singular non-braided monofilament threads such as thread 502, made of, for example, #0 monofilament nylon threads, and a central hub 504. The threads are coupled with hub 504 at one end thereof to form spokes radiating out from central hub 504. Hub 504 is made of nonreactive, sterilizable, degradation-resistant, elastic, non-carcinogenic materials (e.g., nylon, metals, carbon amalgams, silicon and other suitable materials). In Figures 5A-5B, hub 504 exhibits a circular shape. However, hub 504 may exhibit any other suitable shape. The spokes may be equally spaced around the hub or grouped into sectors.

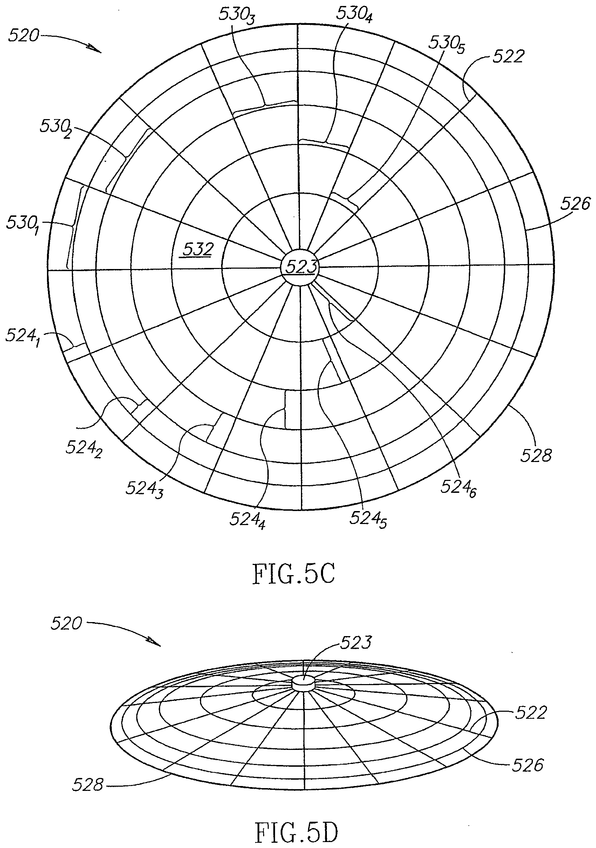

Reference is now made to Figures 5C-5D, which are schematic illustrations of an implantable, incorporable and non-reactive tissue support structure, exhibiting a hub and radiating spokes configuration with concentric rings, generally referenced 520, constructed and operative in accordance with another embodiment of the disclosed technique. Figures 5C is a top view of tissue support structure 520 and Figure 5D is a perspective view of tissue support structure 520. Tissue support structure 520 includes a plurality of threads such as a thread 522, a central hub 523 and at least one concentric ring of thread such as a concentric ring 526. The threads are coupled with central hub 523 at one end thereof and extend radially therefrom, thus forming spokes. The concentric rings are coupled with the spokes at various intervals 524₁, 524₂, 524₃, 524₄, 524₅ and 524₆ around hub 523 and are typically of the same material as the threads. However, other inert materials may be employed. The threads are, for example, lines of #0 monofilament nylon threads. The concentric rings, such as concentric rings 526, serve as stabilizing support for the tissue support structure 520. A perimeter concentric ring 528, coupled with the spokes at the other end thereof, may be of thicker cross-section to increase the stability of tissue support structure 520.

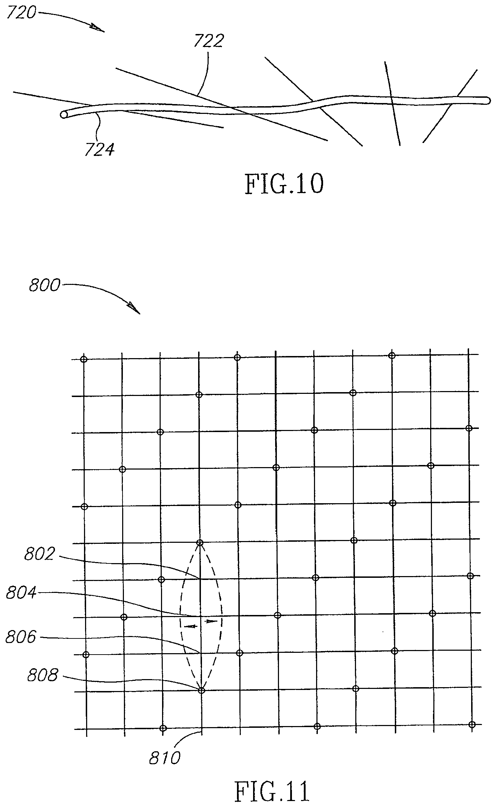

According to one alternative, concentric rings 526 are coupled with the threads (e.g., thread 522) by alternately placing concentric rings 526 over and under the threads. According to another alternative, concentric rings 526 are placed on one side of the threads. All concentric rings may be placed on the same side of the threads, or alternating rings may be placed on alternating sides. Concentric rings 526 may be affixed to the threads, for example, by fusing, soldering or sintering concentric rings 526 to the threads as further explained with reference to Figure 10. A various number of concentric rings may be place at various intervals (e.g., 524₁ and 524₂) therebetween. In general, the dimensions of the resulting spaces, such as space 532, are within pre-determined upper limits. For example no space shall exhibit dimensions greater than 0.5×1.0 cm or 1.0×2.0 cm so as to prevent substantial tissue herniation. The outermost concentric ring, which is also the perimeter of tissue support structure 520, exhibits a diameter such as 18 cm, 20 cm, 30 cm or any other suitable diameter.

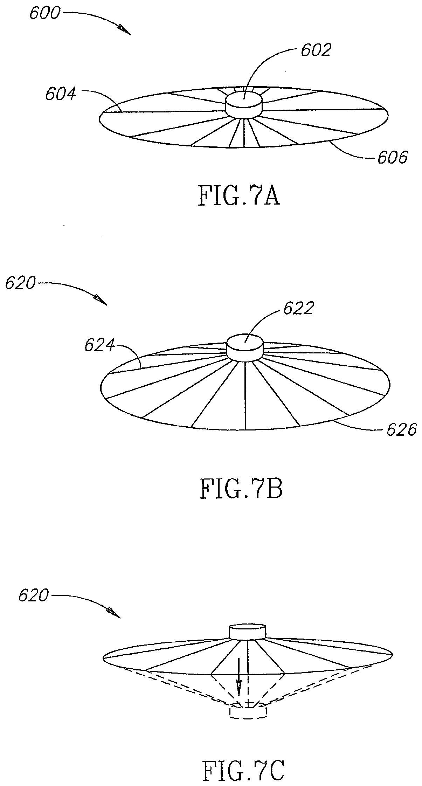

The hub and radiating spokes with concentric rings embodiment may be floppy (i.e., with low tautness) like a spider-web, having increased compliance with abdominal wall forces. Alternatively, a tissue support structure according to the hub and radiating spokes with concentric rings embodiment may be substantially taut and rigid. Tautness may not be uniform throughout the structure. Floppiness and tautness are determined by the lengths of the spokes in relation to the radius of the stabilizing perimeter structure (as described below in Figures 7A-7C regarding invertible convexity) and by the tautness of the inter-spoke intervals of the concentric rings (e.g., 530₁, 530₂, 530₃, 530₄ and 530₅). In all cases, the intersection density of the hub and radiating spokes with concentric rings tissue support structure is small relative to known in the art meshes.

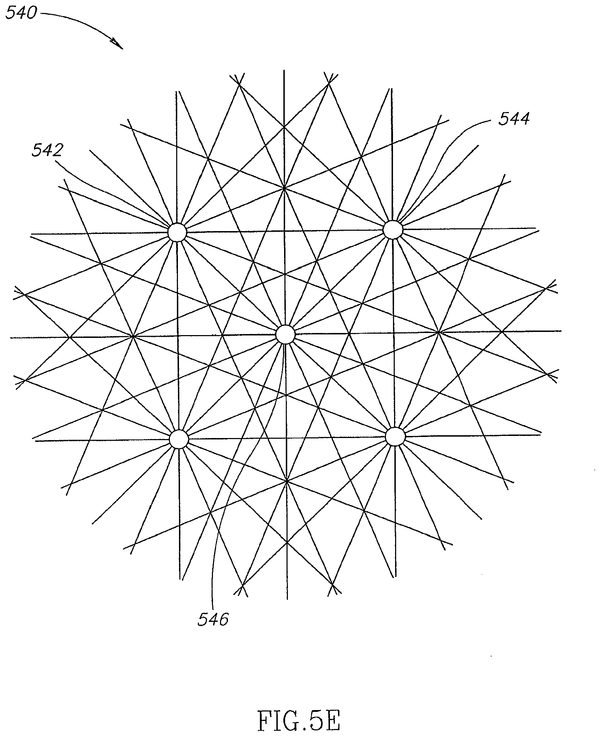

A plurality of tissue support structures exhibiting the hub and radiating spokes configuration, such as described in conjunction with Figures 5A-5B, may be combined to form a tissue support structure array. Reference is now made to Figure 5E, which is a schematic illustration of an implantable, incorporable and non-reactive tissue support structure array made of a plurality of hub with radiating spokes tissue support elements, generally referenced 540, constructed and operative in accordance with a further embodiment of the disclosed technique. Tissue support structure array 540 is formed from a plurality of tissue support elements thus creating a tissue support structure array 540. Tissue support structure array 540 is produced by intersecting the spokes of different tissue support structures such as tissue support structures 542, 544 and 546. Each one of tissue support elements 542, 544 and 546 is similar to tissue support structure 500 (Figure 5A). Such a tissue support structure array may have a perimeter (which is circular, elliptical or quadrilateral in shape) whose diameter may typically be either 20 cm or 30 cm across. Concentric rings (not shown) or other shaped structures may be added as necessary to limit the sizes of intervening spaces.

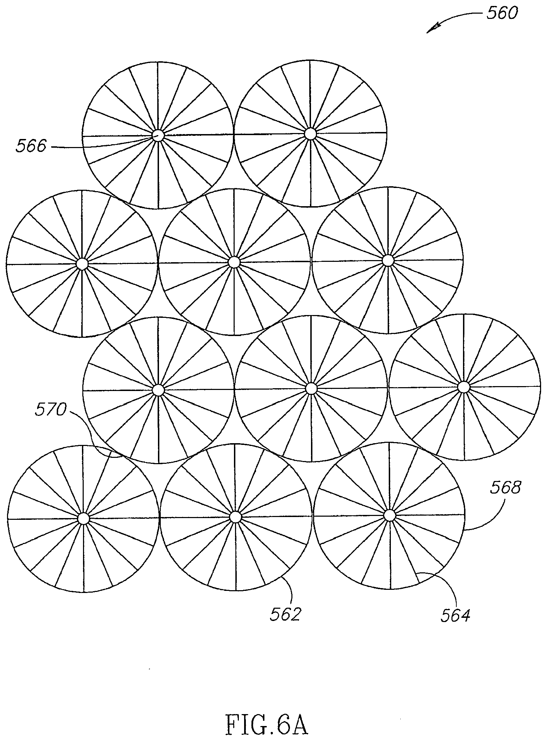

The following description in conjunction with Figures 6A-6B relates to the quilted hubs with radiating spokes configuration. Reference is now made to Figures 6A-6B, which is schematic illustration of an implantable, incorporable and non-reactive tissue support structure array made of quilted hubs with spokes tissue support structures, generally referenced 560 and 580 respectively, constructed and operative in accordance with another embodiment of the disclosed technique. With reference to Figure 6A, tissue support structure array 560 is made of a plurality of tissue support structures, such as tissue support structure 562. Tissue support structure 562 is similar to tissue support structure 500 (Figure 5A). Each tissue support structure includes a plurality of threads such as thread 564, a central hub such as central hub 566 and a stabilizing perimeter structure such as stabilizing perimeter structure 568. The threads are coupled at one end thereof to a respective central hub and at the other end to a respective stabilizing perimeter structure. The respective stabilizing perimeter structures are all coupled at contact points, such as contact point 570, in a "quilted" fashion between the respective stabilizing perimeter structures thereof. It is noted that tissue support structure 560 does not generally need concentric rings due to the smaller diameter of each stabilizing perimeter structure 568. The diameter of all of the stabilizing perimeter structures is typically either 5 cm or 8 cm in diameter, and can be identical or vary throughout the quilt. It is further noted that the diameter of a tissue support structure in quilted hubs with radiating spokes tissue support structure array 560 is generally smaller than the diameter of the single hub and radiating spokes with concentric rings described hereinabove in conjunction with Figures 5A-5D.

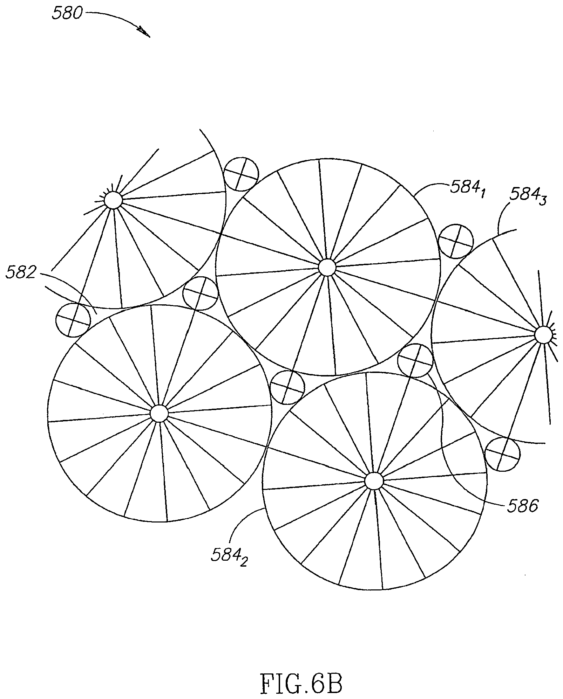

With reference to Figure 6B, tissue support structure array 580 is similar to tissue support structure array 560 (Figure 6A). However, the spaces 582 between the coupled stabilizing perimeter structures, such as stabilizing perimeter structures 584₁, 584₂, 584₃ may be filled by smaller tissue support structure such as tissue support structure 586, to limit the size of any open spaces 582 (e.g., to less than 1.0 cm in diameter). The diameter of tissue support structure 586 is smaller than the diameter of tissue support structure 584₁ and thus requires fewer spokes to prevent tissue herniation. Alternatively, tissue support structure 586 may include only a ring with spokes (i.e., without a hub). Nevertheless, tissue support arrays 560 and 580 are constructed while maintaining an intersection density to be no larger than 10,000 intersections per 100 cm².

Reference is now made to Figures 7A-7D, which are schematic illustrations of implantable, incorporable and non-reactive tissue support structures exhibiting invertible convexity, generally referenced 600 and 620 respectively, constructed and operative in accordance with a further embodiment of the disclosed technique. Figure 7A refers to tissue support structure 600 and Figures 7B and 7C refer to tissue support structure 620. With reference to Figure 7A, the diameter defined by a hub 602 plus twice the length of a thread 604 is equal to the diameter of stabilizing perimeter structure 606. Thus, hub 602, the threads and circular stabilizing perimeter structure 606 are all located on the same plane. With reference to Figures 7B and 7C, the diameter defined by hub 622 plus twice the length of a thread 624 is larger than the diameter of stabilizing perimeter structure 626. Thus, hub 622 and stabilizing perimeter structure 626 can be located in different planes. This results in hub 622 and the threads such as thread 624 being slightly convex in relation to the plane of stabilizing perimeter structure 626. Hub 622, and thus the convexity, alternates between the two sides of the plane defined by stabilizing perimeter structure 626 in response to the physiologic forces of the abdominal wall, and is referred to herein as "invertible convexity" (Figure 7C).

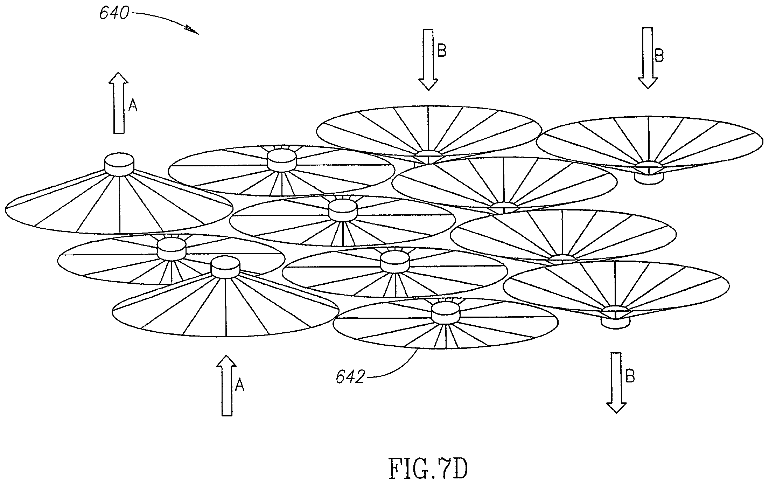

Each tissue support structure of the tissue support structure array 560 described hereinabove in conjunction with Figure 6A, may exhibit an invertible convexity as described above. With reference to Figure 7D, tissue support structure array 640 is constructed from a plurality of tissue support structures, such as tissue support structure 642 which is similar to tissue support structure 620 (Figures 7B and 7C). Due to the invertible convexity of each tissue support structure in tissue support structure array 640, the surface of tissue support structure array 640 readily responds to varying physiologic forces simultaneously acting there upon. For example, these forces may press out in one region (e.g., as demonstrated by arrows A), and pull in (e.g., as demonstrated by arrows B), in another region. Such forces are applied normally over the abdomen wall, and normally cause the abdominal wall to undulate subtly. Tissue support structure array 640 with invertible convexities is thus designed to allow the post-repair abdominal wall to respond to biologic stresses by undulating normally and thus avoiding the "stiff abdomen" syndrome. The size of each stabilizing perimeter structure 642 may exhibit several sizes, for example, 5 cm or 8 cm. The overall size of the tissue support structure array 640 may be, for example, 20×20 cm or 30×30 cm, though it can be made smaller or larger. The individual circles are typically identical in size, but need not be necessarily.

As mentioned above, the tissue support structure according to the disclosed technique may be structurally stabilized. Various stabilizing techniques may be employed. The threads of tissue support structures according to the disclosed technique may be affixed without the addition of a separate stabilizing structure or structures (e.g., perimeter structures), for example, by affixing threads to each other at their intersections (e.g., as described with reference to Figure 3B). Singular monofilament nylon threads can be fused or molded to other threads at right-angles in an end-to-side manner (Figure 3B), to produce an essentially flat grid. Existing manufacturing processes for producing such grid-like structures may be applied to #0 monofilament nylon thread. Threads may be affixed at intersections thereof in additional ways, for example, by sintering. Also, when different threads made of different materials are used, the temperature during the sintering process may be gradually increased to differentially including the melting points of some materials used, and thus to selectively affix the threads. Another example may employ reversible brief nylon dissolution processes, such as by heat or chemicals, which leave the singular monofilament thread unchanged except for a 'soldering' effect at the precise points of contact. According to another example, selected points of contacts are individually soldered by a hand-held or automated electrode (i.e., when such affixing is sufficient) or by employing plastic adhesives resistant to both sterilization and the physiologic environment. Other affixing options, such as fusion by molding, are possible.

Furthermore, stabilization of singular monofilament threads to hubs and concentric rings (for example, Figure 5C) may also be done by dissolution processes similar to those described above or by physically embedding the nylon threads into the nylon hubs using existing manufacturing processes, or by initial molding. Concentric rings may be stabilized with the threads by affixing contact points as described above with reference to stabilizing a thread to another thread.

When a perimeter structure is added to the tissue support structure, the perimeter structure generally defines the shape of the tissue support structure in addition to providing stabilization. Reference is now made to Figures 8A-8D, which are schematic illustrations of different exemplary cross-sections of a stabilizing perimeter structure constructed and operative in accordance with another embodiment of the disclosed technique. This perimeter structure may be for example structure 204, in Figure 2A, structure 262 in Figure 2D, or structure 528 in Figure 5C. Figure 8A depicts a circular cross-section, Figure 8B depicts a triangular cross-section, Figure 8C depicts a square cross-section and Figure 8D depicts a rectangular cross-section. The stabilizing perimeter structures may be hollow or solid. Also, depending on the function and form of the tissue support structure, the stabilizing perimeter structure may be a #0 thread of 0.35mm cross-section (e.g., rollable for laparoscopy), or, for example, exhibit a 1.0 cm × 1.0 cm cross section. The stabilizing perimeter structure may exhibit different dimensions, for example, between 15 cm and 40 cm in diameter for circles or in side-length for quadrilaterals.

Stabilizing perimeter structures are typically made of synthetic and nonreactive, sterilizable, resistant to degradation, flexible, non-breakable under stress and non-carcinogenic materials such as nylon, metals, carbon amalgams and silicon. Stabilizing perimeter structures should also be suitable for attaching singular monofilament threads (e.g., #0 nylon threads) thereto by, for example, soldering or initial molding. Reference is now made to Figures 9A and 9B which are schematic illustrations of a section, generally referenced 700, of an implantable, incorporable and non-reactive tissue support structure constructed and operative in accordance with a further embodiment of the disclosed technique. Section 700 includes a plurality of threads, such as a thread 702, coupled with a stabilizing perimeter structure 704. In Figure 9A, the thickness of stabilizing perimeter structure 704, which exhibits a rectangular cross-section, is equal to the diameter of monofilament thread 702 coupled therewith. Figure 9B depicts an enlarged view of section 700. Coupling threads, such as thread 702, to rectangle stabilizing perimeter structure 704 of similar thickness generally minimizes the presence of 'nooks and crannies' at a coupling point 706. It is noted that the cross-section of the stabilizing perimeter structure may be non-rectangular (e.g., oval), while maintaining the aforementioned principal of the coupling point (i.e., maintaining a similar thickness). As mentioned, the stabilizing perimeter structure may be molded in one piece with threads attached.

In some applications, stabilizing perimeter structures should be rigid enough to provide overall structural integrity to the tissue support structure and yet be flexible enough to respond to abdominal wall kinetics, and even to be rolled up (with neither loss of 'shape memory' nor breaking), for example, for use in laparoscopic surgery.

Separate ring-like stabilizing structures, such as concentric ring 526 (Figures 5C-5D), may be layered onto, or woven into, the body of the tissue support structure. Reference is now made to Figure 10 which is a schematic illustration of a section, generally referenced 720, of an implantable, incorporable and non-reactive tissue support structure, constructed and operative in accordance with another embodiment of the disclosed technique. In Figure 10, a ring stabilizing structure 724 is woven through threads such as a thread 722 of tissue support structure 720. Ring stabilizing structures 724 may exhibit various cross-sections (e.g., round, semicircular, or flat) to minimize nooks and crannies. Ring stabilizing structure 724 may be affixed to the tissue support structure by any of the above described methods (e.g., soldering or molding).

A matrix tissue support structure such as described above in conjunction with Figure 3B, is relatively independent of size or shape considerations when affixed firmly (e.g., molded) at each intersection of the threads. Thus, such a structure may be intra-operatively contoured to any shape and size, without reducing the stability thereof.

However, in the matrix embodiments described above in Figures 3C and 3D, the manufacturing step of weaving or layering the intersecting threads does not confer adequate stability. Affixing intersections to increase stability may be done as described above by the chemical or thermal "soldering" of intersections either universally by automotive processes (e.g., sintering), or individually by manual techniques. This results in a ready-made sheet of a network with "infinite" integral stability, which is also independent of size and shape considerations. The manufacturing process may be supplemented, for example, by affixing some or all of the intersections or by addition of separate perimeter stabilizing structures or both.

The matrix tissue support structure may be attached to a stabilizing perimeter structure. The threads of the matrix may be embedded in, manufactured with or slung around the stabilizing perimeter structure. When a stabilizing perimeter structure is employed, the stability of the tissue support structure is related to the size and shape of the stabilizing perimeter structure. Employing a stabilizing perimeter structure precludes intra-operative contouring, but provides a ready-to-use structure.

In the hubs with spokes and rings configuration, stabilization includes affixing the singular monofilament threads both into the central hub and into the perimeter ring structure. In the former (i.e., coupling with the central hub), affixing may be done by embedding or molding, as part of the initial manufacturing process. In the latter (coupling with the perimeter ring structure), affixing may be done by techniques identical to those described with reference to Figure 9A and 9B. For stabilization of the concentric rings which are woven or laid onto the radiating spokes, affixing the contact point may be done between the two elements, as described above with reference to Figure 10.

The quilted tissue support structure (Figures 6A and 6B) may be obtained by coupling one perimeter structure to another by brief chemical or thermal dissolution, or by selective manual soldering of contact points, or by the application of plastic adhesives. The space-filling miniature rings-with-spokes may similarly be affixed in place.

One of the advantages of the tissue support structures according to the disclosed technique is the compliance to the physiological and the pathological strains and loads of the human body, such as those resulting from the active kinetics of the abdominal wall muscles or from passive responses of the abdominal wall to changes in intra-abdominal pressure. Increasing compliance in the tissue support structures according to the disclosed technique gives better dispersion of locally acting forces. This improves the adaptability of the tissue support structure to the host body and thus, for example, minimizes mesh disattachment with hernia recurrences, as well as post-implantation pain syndromes relative to known in the art meshes.

The increased compliance of the tissue support structures according to the disclosed technique is due, inter alia, to the tension relation of the singular monofilament threads to the stabilizing perimeter structure. Thus, for example, when threads are stretched between a central hub and surrounding perimeter structure, compliance can depend on the tautness of the threads or on an invertible convexity design as described above.

When nylon threads are independently stretched across a perimeter structure, their compliance is inversely related to their tautness. Thus, the compliance of the structure may be adjusted by adjusting the tautness. When only a separate perimeter structure is used for matrix stabilization, producing a "closed" matrix, the singular monofilament threads may be stabilized solely by their affixing to the perimeter. This leaves the threads of the body of the matrix potentially very compliant, depending on the tautness of their 'stretch' across the overall surrounding perimeter structure. This similarly applies to the parallel lines configuration when stretching parallel threads between the perimeter structures.

However, increasing thread compliance is more difficult to achieve when a perimeter structure is not present, as in "open" matrices. In such configurations, which do not include a perimeter stabilizing structure, the stabilization of the tissue support structure depends on affixing the thread intersections. In these configurations, the compliance of the tissue support structure may be controlled by utilizing the principle that compliance is inversely related to the number and distribution (i.e., over the area of the matrix) of affixed intersections. Decreasing the number of affixed intersections distributed over the entire area of the matrix, results in increasing the number of non-affixed intersections and thus in a more compliant structure.

Reference is now made to Figure 11, which is a schematic illustration of a tissue support structure exhibiting the matrix configuration, generally referenced 800, constructed and operative in accordance with a further embodiment of the disclosed technique. Tissue support structure 800 is similar to tissue support structure 320 (Figure 3A). Tissue support structure 800 includes three unfixed intersections, such as intersections 802, 804 and 806, for each one affixed intersection such as intersection 808, along the length of any thread, such as a thread 810. In Figure 11, affixed intersections are depicted by bold dots. The arrows and dotted lines show the direction and extent of the potential lateral motion of thread 810 (i.e., which represents the compliance thereof) in a representative segment affixed as described.

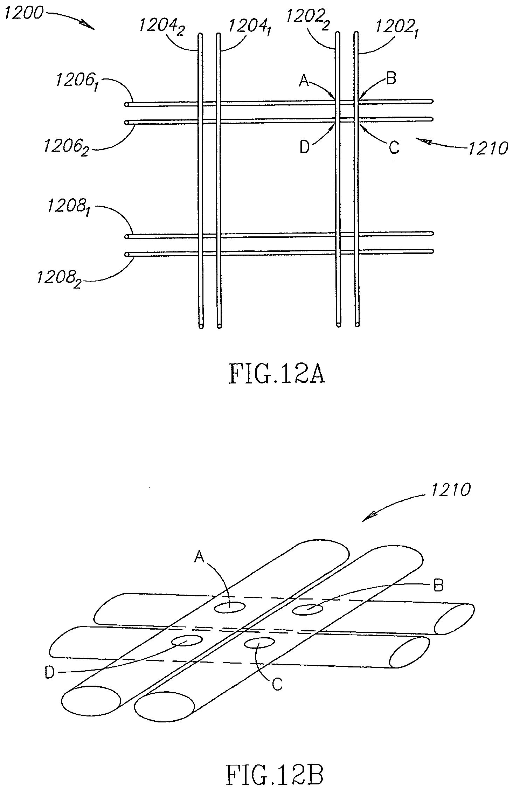

According to embodiments of the invention a network may be constructed using "twinned" (i.e., doubled) adjacent, substantially in loose parallel contact, singular monofilament threads. Reference is now made to Figures 12A - 12C which are schematic illustrations of a tissue support structure, generally referenced 1200 constructed and operative in accordance with another embodiment of the disclosed technique. Tissue support structure 1200 includes twinned threads 1202₁ and 1202₂ which lie parallel to other twinned threads 1204₁ and 1204₂ and intersect twinned threads 1206₁ and 1206₂ and 1208₁ and 1208₂. Each of twinned threads 1202₁ and 1202₂, are in loose parallel contact with each other. Similarly twinned threads 1204₁ and 1204₂, twinned threads 1206₁ and 1206₂ and twinned threads 1208₁ and 1208₂ are also in loose parallel contact with each other. When a single thread intersects another single thread there is only one point of contact between them, referred to above as an "intersection" for potential affixing. When twinned threads (e.g., 1202₁ and 1202₂) intersect other twinned threads (e.g., 1206₁ and 1206₂) there results a group of intersecting twinned threads, such as intersection group 1210, which includes four points of contact A, B, C and D, any one of which can potentially be affixed. It is noted that each of the contact points can be uniquely defined by the position thereof in the group of intersecting twinned threads, relative to the orientation of the group to the overall matrix (e.g., D lower left corner of the group, B upper right corner).

Figure 12B depicts an enlarged view of intersection group 1210 (Figure 12A). When only one of the four contact points (e.g., A) is affixed, the three other contact points (B, C and D) remain free and mobile, resulting in an increase in the local compliance of the threads. Conversely, affixing more of the contact points of intersection group 1210 decreases the compliance (i.e., the mechanical response of the threads of the tissue support structure to a force applied thereon), which provides greater matrix stability. Thus, the extent of group fixation may be exploited to achieve an overall compliance and stability of the entire tissue support structure, which may simultaneously reduce post-implantation pain and improve barrier or reinforcement functions.

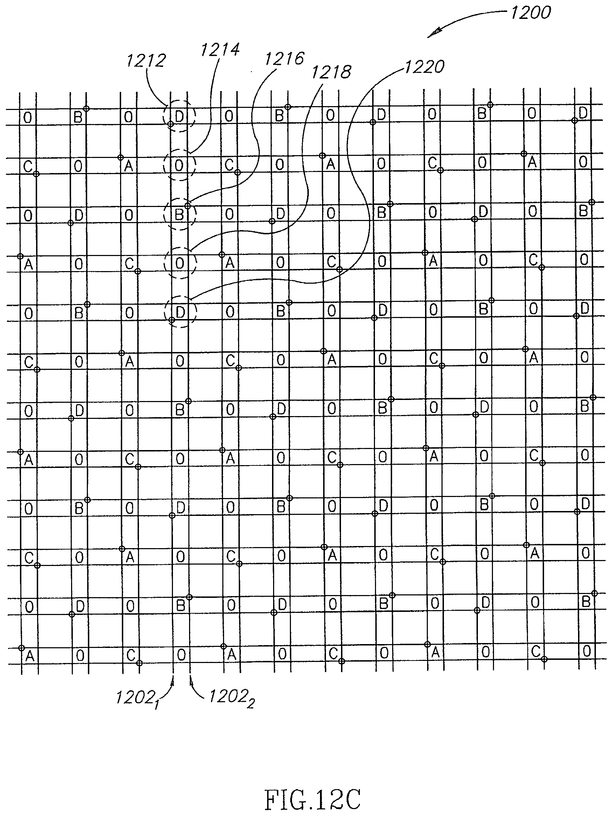

Figure 12C schematically illustrates how desired compliance may be achieved. In Figure 12C, each intersection group (e.g., intersection groups 1212, 1214, 1216, 1218 and 1220) is labeled with one of the letter "A", "B", "C", or "D", corresponding to the affixed intersection position, as defined in Figure 12B or with "0" when none of the intersection positions in group is affixed. For each one of the single threads (e.g., 1202₁) of a twinned pair 1202₁ and 1202₂, the thread remains non-affixed for a segment of three intersection groups. In the fourth intersection group, thread 1202₁ is affixed, as represented by a bold dot at position D. Accordingly, for any single thread, there is potential for mobility and compliance. A different thread (e.g., 1202₂) is affixed at a different intersection position (i.e., B) at the fourth intersection group of thread 1202₂. The result is that every second group is affixed, albeit at just one of its four contact points. This increases the stability of tissue support structure 1200 as illustrated in Figure 12C, while increasing the compliance of individual threads. This is depicted by twinned threads 1202₁ and 1202₂ and the groups they form with other twinned threads. In Figure 12C, affixed intersections are depicted by a bold dot and their intersection position is indicated by the letter within each intersection group. A first group 1212 is affixed only at its intersection position D, the following group 1214 is not affixed at all (marked "0"). The next group 1216 is affixed only at its intersection group position B and the fourth group 1218 is not affixed. The sequence along this individual thread then begins again with group 1220. Affixing the threads as described above achieves a desired compliance in an orderly fashion, with the specific position of the affixed contact point (A,B,C,D) within each twinned intersection group being as equally spaced as possible across the network. Thus, no group ever occurs in near proximity to another group with identical affixed contact point. For example, a group with affixed 'upper left' (A) is surrounded by groups with either 'upper right' (B), 'lower right' (C) or 'lower left' (D) fixation, or by groups which are not affixed (0). Thus, groups in which the same contact point is affixed are at the largest possible distance from each other, thus simultaneously increasing compliance while maintaining stability.

In summary, a tissue support structure according to the disclosed technique employs a twinned pair of threads, which intersect other twinned pairs at intersecting groups such that there are at least four uniquely identifiable thread contact point positions in each group which can be selectively affixed to increase compliance and maintain stability.

Each set of twinned threads in tissue support structure 1200 (Figures 12A and 12C), includes a pair of only two threads. However, each set may include three or more adjacent threads. For example, each thread in a set of three adjacent threads is affixed once every ninth intersection with other threads, such that each third intersection group includes at least one affixed contact point. As described above, such a tissue support structure may be designed such that intersection groups in which the same intersection position is affixed are at the largest possible distance from each other. It is, however, noted that more than one intersection position within a single intersection group may be simultaneously affixed. Thus, meshes can be designed with respective compliance and stability according to abdominal wall stresses.

The stability of such a network of compliant twinned threads is particularly relevant and advantageous for use in the abdominal wall where forces acting to either disperse or "bunch up" individual network threads (thus decreasing their barrier function) are resisted by the orderly arrangement of partially affixed complexes, as illustrated above. Nevertheless compliance, which contributes to the reduction in pain, is maintained. Such orderly partial affixing of the threads may be integrated into the network by automated or manual means as described. Affixing the threads randomly may achieve a similar overall stability/compliance ratio and may have manufacturing advantages.

It should be noted that when only single, non-twinned, threads are used, desired matrix compliance and thus matrix stability, can be achieved by simply altering the frequency of affixing of the single thread intersections. Twinned threads, tripled threads and the like, may be used in any of the embodiments described above.

As mentioned, a tissue support structure exhibiting the matrix configuration, affixed (e.g., molded) at all the intersections thereof, has less potential for thread compliance. However, since such matrices still incorporate uniquely wide spaces (e.g., 2 cm in diameter) inherent in the matrix configuration, even a minimal decrease in thread tautness, between the affixed intersections, can substantially increase compliance.

As mentioned above, in a tissue support structure exhibiting the parallel lines configuration, a surgeon has the option of layering one tissue support structure on top of another at different angles during surgery. The final configuration having reinforcing or barrier functions is thus produced during surgery. This may require coupling one stabilizing perimeter structure to another. Accordingly, the stabilizing perimeter structures of the disclosed technique are configured to enable the coupling of one perimeter structure to another to achieve a multilayered device. As described above in conjunction with Figures 8A-8D, a circular or quadrilateral perimeter structure is constructed of a frame whose cross-section has various shapes. Reference is now made to Figures 13A-13G, which are schematic illustrations of cross-sections of various stabilizing perimeter structures, constructed and operative in accordance with a further embodiment of the disclosed technique. With reference to Figure 13A, a frame 1300 exhibiting a substantially rectangular cross-section, has an elevation or ridge 1302 at the center of the upper side thereto, with corresponding indentation 1304 of the underside thereof, allowing stable coupling of a similar frame 1306 with frame 1300. With reference to Figure 13B, the cross-section of each of frames 1320, 1322 and 1324 exhibit an inverted V-shape to allow the stable coupling of frames.

With Reference to Figure 13C, small grooves such as groove 1344 engraved across the top of frame 1340 at predetermined intervals. The small grooves such as groove 1344 are designed to hold a suture 1346 which passes around frames 1340, 1342 and 1348. The suture typically passes simultaneously through the underlying tissues and securely tightens into groove 1344, thus simultaneously securing the frames one to the other, and to the tissues.

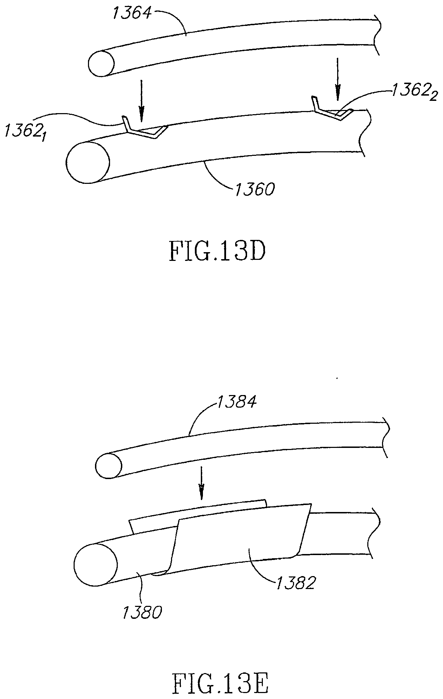

With reference to Figure 13D a frame (i.e., a stabilizing perimeter structure) 1360, which exhibits a circular cross-section, is manufactured with coupling elements such as clips 1362₁ and 1362₂. Another frame such as frame 1364 may be inserted into clips 1362₁ and 1362₂ thus coupling the two frames.

With reference to Figure 13E a frame 1380, which exhibits a circular cross-section, is manufactured with a coupling element such as a housing 1382. Another frame, such as frame 1384 may be inserted into housing 1382 thus coupling the two frames. It is noted that coupling elements are not limited to frames which exhibit a circular cross-section. Such coupling elements may be manufactured with frames which exhibit other cross-sections such as those described above in conjunction with Figures 8A-8D.

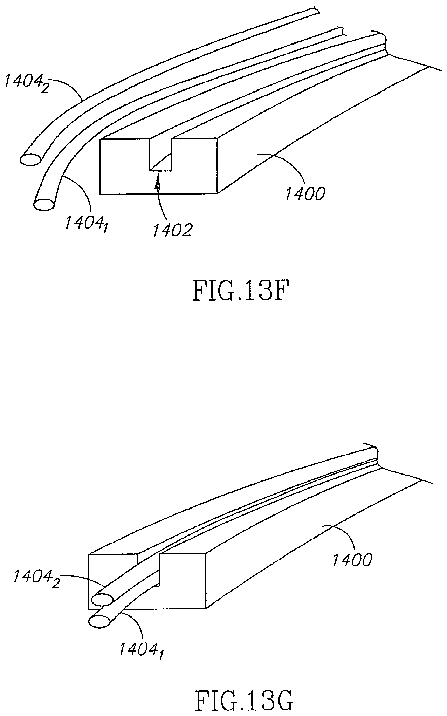

With reference to Figures 13F and 13G, frame 1400, exhibiting a generally rectangle-cross section, has a groove 1402 running longitudinally along the upper surface thereof. Other tissue support structures with respective stabilizing perimeter structures such as stabilizing perimeter structures 1404₁ and 1404₂, exhibiting a narrower cross-section than groove 1402, may be inserted securely into groove 1402, for example, one on top of the other as illustrated in Figure 13F, and are thus layered on as illustrated in Figure 13G.

According to the disclosed technique, using a singular non-braided monofilament thread reduces the thread intersection density (i.e., the number of thread intersections per 100 square centimeters) and increases the effective porosity (i.e., either existing spaces allowing for unimpeded tissue ingrowth or spaces created during the ingrowth of the tissue) relative to known in the art meshes. This results in substantially reducing the tissue reaction to the structure while maintaining the incorporability thereof. As mentioned above, the relatively high intersection density (e.g., 250,000 intersections per 100 cm²) of woven or entangled braided monofilament threads and multifilament threads in known in the art fiber based meshes results in complicated tissue reactions (e.g., chronic pain and infection) in these known in the art meshes.

Tissue support structures according to the disclosed technique relate to networks exhibiting the same non-reactivity as known in the art smooth solid sheets, but, as opposed to known in the art smooth solid sheets, are also incorporable. Moreover, the tissue support structures according to disclosed technique provides an inert and permanent "scaffolding", around which normal healing processes occur to strengthen the abdominal wall. Furthermore, the non-reactive incorporable "scaffolding" can be manipulated to spatially extend normal healing processes, which is useful, for example, in the definitive closure of wide-open catastrophic abdomens.

It is noted that the tissue support structure according to the disclosed technique limits tissue reactions to an extent equivalent to a fully dissolved biologic mesh. However, as opposed to biological meshes, tissue support structures according to the disclosed technique are also permanent and thus can prevent hernia recurrence. It is also noted that the production cost of a tissue support structure according to the disclosed technique is currently lower than the production cost of a known in the art biological mesh. However, when necessary, biologic meshes, biological threads or absorbable coatings may be incorporated into the tissue support structures of the disclosed technique.

The non-braided monofilament threads of a tissue support structure according to the disclosed technique may also be coated by, or otherwise conjugated with drug delivery systems and cell micro-carriers which have known skin-engineering applications (e.g., skin formation). Drugs (e.g., epidermal growth factors) and cells (e.g., keritanocytes and fibroblasts) with the ability to increase granulation and epithelialization can thus be used to increase skin formation across the area covered by the tissue support structure. Examples of relevant drug delivery systems are dextran-base hydrogels, nano-scaled poly (epsilon-caprolactone) gelatin fibrous scaffold, and injectable forms of silicon-resin particles. Examples of cell micro-carriers are dextran gelatin particles and plastic particles made of polyethylene or polystyrene. As mentioned, stabilizing structures and the thread sections of a tissue support structure according to the disclosed technique may be hollow or fenestrated, thus allowing their impregnation with the skin-engineering drugs or cells.

A desirable place to implant a tissue support structure is on the highly accessible outer surface of the abdominal muscles (i.e., onlay placement). This is precluded with known in the art meshes since, as described above, they may become infected in the onlay position. However, the tissue support structures according to the disclosed technique prevent chronic infection, allowing full utilization in the onlay position. Thus, tissue support structures according to the disclosed technique may be applied during surgery, for example, for routine hernia prophylaxis. In summary, tissue support structures according to the disclosed technique provide a new incorporable and non-reactive solution for hernia repair.

Tissue support structures according to the disclosed technique may similarly be used as non-reactive and incorporable tissue implants in other medical procedures, for example, esophageal replacement, chest wall reconstruction, large area skin graft substrate, pelvic soft-tissue reconstruction (e.g., pelvic floor slings) and conduit reconstruction (e.g., intestinal and ureteral stomae) and management (e.g., treatment of prolapse), acetabular replacement and cement restriction in orthopedic surgery, as well as for abdominal wall reconstruction as described in detail herein above. For some uses, a smaller diameter monofilament nylon thread (e.g., less than 0.2 mm in diameter) may be employed to allow more closely arrayed threads or increased flexibility without losing non-reactivity and incorporability, as for example, to allow the matrix configuration to be rolled into a flexible cylindrical shape to provide a permanent artificial surface for esophageal replacement.

It will be appreciated by persons skilled in the art that the disclosed technique is not limited to what has been particularly shown and described hereinabove. Rather the scope of the disclosed technique is defined only by the claims, which follow.

## Claims

1. An implantable tissue support structure (320) for supporting musculoaponeurotic tissue, said tissue support structure (320) being formed by a plurality of monofilament thread sections (324), said monofilament thread sections (324) defining a surface having a plurality of open spaces (328), the tissue support structure (320) **characterized in that** said surface comprises less than 10,000 thread intersections (326) per one-hundred square centimeters, said thread intersections (326) being defined as a crossing of two of said monofilament thread sections (324), **characterized in that** per one-hundred square centimeters of said surface each of at least four of said open spaces (328) has an area greater than one-hundred square millimeters and up to three thousand square millimeters.

## Patentansprüche

1. Implantierbare Gewebestützstruktur (320) zum Stützen von muskuloaponeurotischem Gewebe, wobei die Gewebestützstruktur (320) gebildet wird durch eine Mehrzahl von monofilen Fadenkreuzungen (324), wobei die monofilen Fadenkreuzungen (324) eine Oberfläche definieren, die eine Mehrzahl von offenen Flächen umfasst (328), wobei die Gewebestützstruktur (320) **dadurch gekennzeichnet ist, dass** die Oberfläche weniger als 10000 Fadenkreuzungen (326) pro einhundert Quadratzentimetern umfasst, wobei die Fadenkreuzungen (326) definiert sind als eine Überschneidung von zwei monofilen Fadenkreuzungen (324), **dadurch gekennzeichnet, dass**
pro einhundert Quadratzentimetern der Oberfläche jede von wenigstens vier der offenen Flächen (328) ein Gebiet mit einer Größe von mehr als einhundert Quadratmillimetern bis zu dreitausend Quadratmillimetern umfasst.

## Revendications

1. Structure de support de tissu implantable (320) pour supporter un tissu musculoaponévrotique, ladite structure de support de tissu (320) étant formée d'une pluralité de sections de fil monofilament (324), lesdites sections de fil monofilament (324) définissant une surface ayant une pluralité d'espaces ouverts (328), la structure de support de tissu (320) étant **caractérisée en ce que** ladite surface comprend moins de 10 000 intersections de fil (326) pour cent centimètres carrés, lesdites intersections de fil (326) étant définies comme un croisement de deux desdites sections de fil monofilament (324), **caractérisée en ce que** pour cent centimètres carrés de ladite surface, chacun d'au moins quatre desdits espaces ouverts (328) a une surface supérieure à cent millimètres carrés et pouvant atteindre trois cent millimètres carrés.
